# EUROPEAN PATENT APPLICATION

(11) **EP 4 062 956 A1**
(43) Date of publication of application: **28.09.2022**
(21) Application number: 20889280.2
(22) Date of filing: 20.11.2020
(51) Int. Cl.: A61M 5/142, A61J 15/00

(54) **HOLDING APPARATUS FOR LIQUID INFUSION PUMP AND LIQUID INFUSION DEVICE COMPRISING SAME**

(30) Priority: 20.11.2019 CN 201922016371 U
(71) Applicant: Fresenius Kabi (Nanchang) Co., Ltd., Nanchang, Jiangxi 330013 (CN)
(72) Inventor: XU, Jian, Nanchang, Jiangxi 330013 (CN); XIA, Ningbo, Nanchang, Jiangxi 330013 (CN); LAU, Fat Kit, Nanchang, Jiangxi 330013 (CN)
(74) Representative: Fresenius Kabi Deutschland GmbH
(86) International application number: PCT/CN2020/130360
(87) International publication number: WO 2021/098816

(57) **Abstract**

The present disclosure relates to a holding device for a liquid infusion pump and a liquid infusion device including the holding device. The holding device for the liquid infusion pump includes a base and a holding member. The base includes a housing, to which the liquid infusion pump is detachably attached; a power supply adapter arranged in the housing; a power supply interface configured to electrically connect an external power supply with the power supply adapter; and electronic interfaces configured to electrically connect with the liquid infusion pump. The holding member is connected with the base and is configured to detachably position the holding device in a predetermined position in service environment. The holding device for the liquid infusion pump and the liquid infusion device including the holding device according to the present disclosure have optimized configuration and higher cost-effectiveness, and are simple in structure and easy to operate, and can better prevent misoperation disassembly, and further improve water-tightness of the housing of base, thereby improving the use reliability of devices.

## Description

The present application claims priority to Chinese patent application No. 201922016371.X filed with the China National Intellectual Property Administration on November 20, 2019 and titled "HOLDING DEVICE FOR LIQUID INFUSION PUMP AND LIQUID INFUSION DEVICE INCLUDING THE HOLDING DEVICE". The entire contents of the above patent application are incorporated herein by reference.

### FIELD

The present disclosure relates to the field of medical instruments, and in particular, to a holding device for a liquid infusion pump and a liquid infusion device including the holding device.

### BACKGROUND

The contents in this section provide background information relating to the present disclosure, which does not necessarily constitute the prior art.

The liquid infusion pump (for example nutrient solution infusion pump) is a medical device commonly used in the medical field, which is configured to infuse nutrients and medicaments into a patient. For stable holding and real-time monitoring of the liquid infusion pump, the liquid infusion pump is further equipped with a holding device. At present, the common holding device includes: a base configured to connect and support the liquid infusion pump; a holding member configured to fix the base supporting the liquid infusion pump to a predetermined position in service environment; and an external power supply device configured to supply power to the base and the liquid infusion pump. The external power supply device includes a power supply adapter, of which one end connects to the base and the other end connects to the external alternating current power supply, and which converts the external alternating current into direct current and supply the direct current to the base.

In practice applications, it has been found that such power supply adapter is arranged outside the housing of the base and loosely structured, which results in a number of components of the liquid infusion system and complex usage. In addition, the plug of the power supply adapter needs to be specially customized to match the external power supply interfaces of different types. The power supply adapter cannot be used once the plug is damaged, and thereby the liquid infusion pump cannot be used. It is necessary to improve the device due to the high cost of replacement and maintenance. In another aspect, the design for the housing of the base in the prior art also needs to be improved in terms of preventing accidental disassembly, waterproof and cable fixing.

Therefore, it is desired to provide a holding device for the liquid infusion pump and a liquid infusion device including the holding device that can solve the above and other problems and improve the above aspects.

### SUMMARY

An object of the present disclosure is to provide a holding device for a liquid infusion pump which has a compact structure.

Another object of the present disclosure is to provide a holding device which cannot be easily disassembled by the user.

Still another object of the present disclosure is to provide a holding device of which the housing is waterproof.

Yet another object of the present disclosure is to provide a holding device that provides a safety holding for connection cables.

According to one aspect of the present disclosure, a holding device for a liquid infusion pump is provided, the holding device comprising
a base and a holding member, wherein
the base comprises
a housing, to which the liquid infusion pump is detachably attached;
a power supply adapter arranged in the housing;
a power supply interface configured to electrically connect an external power supply with the power supply adapter; and
electronic interfaces configured to electrically connect with the liquid infusion pump; and
the holding member is connected with the base and is configured to detachably position the holding device in a predetermined position in service environment.

According to a preferred embodiment of the present disclosure, the electronic interfaces comprise first electronic interfaces configured to supply power to the liquid infusion pump.

According to a preferred embodiment of the present disclosure, the electronic interfaces comprise second electronic interfaces configured to carry electronic communication between the base and the liquid infusion pump.

According to a preferred embodiment of the present disclosure, the housing comprises a first housing portion and a second housing portion, the first housing portion and the second housing portion are configured as a drawer-type insertion configuration, and an edge outer wall of the second housing portion is lapped jointed to an inner wall of the first housing portion to form a seal.

According to a preferred embodiment of the present disclosure, the first housing portion is detachably connected to the second housing portion by snap engagement or screw connection, and the first housing portion and the second housing portion are configured such that, during using, the first housing portion is always located above the second housing portion in a gravity direction.

According to a preferred embodiment of the present disclosure, the housing further comprises a side cover, which is fixed to the housing by a side cover screw, and the side cover is configured to cover a first screw that connects the first housing portion and the second housing portion.

According to a preferred embodiment of the present disclosure, the housing further comprises a cover plate fixed to the housing by bonding or snapping, and the cover plate is configured to cover the side cover screw.

According to a preferred embodiment of the present disclosure, the second housing portion comprises an insertion sheet, which is inserted into the first housing portion and fixed to the first housing portion by a second screw.

According to a preferred embodiment of the present disclosure, the holding member is positioned on an outer surface of the first housing portion and fixed to the first housing portion and the insertion sheet by the second screw.

According to a preferred embodiment of the present disclosure, the housing comprises cable holding members protruding from the housing to form hook-shaped members, and the cable holding members are configured to fix cables and prevent the cables from loosening and displacement.

According to another aspect of the present disclosure, a liquid infusion device is provided, comprising
a holding device for a liquid infusion pump as stated above; and
the liquid infusion pump, which is detachably held by the holding device, wherein,
the holding device comprises a base, the base and the liquid infusion pump are fixedly connected to each other by snap-engagement, and electronic interfaces on the base are electrically connected with corresponding electronic interfaces on the liquid infusion pump in a one-to-one correspondence.

In conclusion, by integrating the adapter into the base of the holding device, the base and the liquid infusion pump could be supplied power via only common power supply wires directly connected to the base, and the operation of device may be ensured by only replacing power supply wires. Therefore, the holding device for the liquid infusion pump and the liquid infusion device including the holding device according to the present disclosure have an optimized configuration and higher cost-effectiveness, and have a simple structure, which is easy to implement. The configuration of the housing of the base is optimized such that the housing can be better prevented from being disassembled due to misoperation, and further improves water-tightness of the base of the housing, thereby improving the use reliability of the holding device,.

### BRIEF DESCRIPTION OF THE DRAWINGS

The foregoing and additional features and characteristics of the present disclosure will become clearer according to the following detail description with reference to the drawings. The drawings are only as examples, which are not necessarily drawn to scale. The same reference numeral indicates the same component in the drawings. In the drawings:
Figure 1 shows a top view of a holding device for a liquid infusion pump according to one preferred embodiment of the present disclosure.
Figure 2 shows a bottom view of the holding device for the liquid infusion pump in Figure 1.
Figure 3 shows an exploded view of the holding device for the liquid infusion pump in Figure 1.
Figure 4a to Figure 4e show the state when the holding device for the liquid infusion pump in Figure 1 is used together with the liquid infusion pump.
Figure 5a to Figure 5d show a further exploded view of the holding device for the liquid infusion pump in Figure 1.
Figure 6 shows the state when cables are inserted into the holding device for the liquid infusion pump in Figure 1.
Figure 7a and Figure 7b respectively show a top view and a bottom view of the holding device for the liquid infusion pump according to another preferred embodiment of the present disclosure.

**Reference numerals**

| | | | | | |
|---|---|---|---|---|---|
| liquid infusion pump | 2; | holding device | 1,1'; | base | 11,11'; |
| holding member | 13; | housing | 112; | | |
| first housing portion | 1122; | second housing portion | 1124; | adapter | 15; |
| circuit board | 17; | power supply interface | 152,152'; | | |
| first electronic interfaces | 172,172'; | second electronic interfaces | 174; | | |
| communication interface | 176; | retaining member | 132; | | |
| adjusting handle | 134; | insertion sheet | 1129; | bottom base | 1321; |
| retaining arm | 1323; | thread segment | 1341; | arrow A; | |
| support | 1140; | convex portion | 1121; | | |
| concave portion | 21; | electronic interfaces | 23; | locking rod | 1123; |
| protrusion | 1125; | side cover | 1126; | | |
| side cover screw | 1127; | cover plate | 1128; | first screw | 1131; |
| thread hole | 1120; | central hole | 1320; | third screw | 1133; |
| inner wall | 1130; | power supply cable | 141; | | |
| communication cable | 142; | cable holding member | 144; | | |
| second screw | 1132 | | | | |

### DETAILED DESCRIPTION

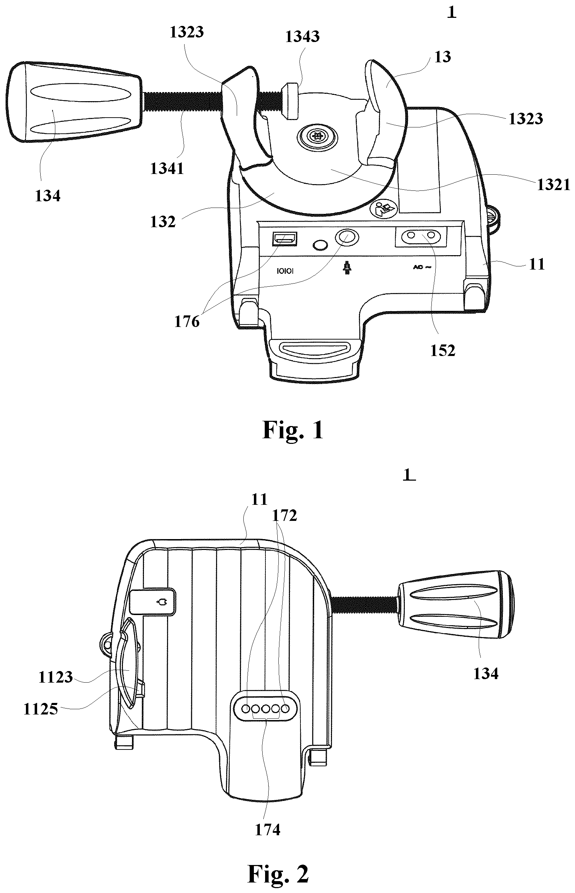

The present disclosure relates to a holding device for a liquid infusion pump and a liquid infusion device including the holding device, and in particular, to a holding device for the medical liquid infusion pump and a medical liquid infusion device including the holding device. The preferred embodiment of the present disclosure will now be described in detail with reference to the Figures 1 to 7b. The following descriptions are only exemplary and are not intended to limit the present disclosure and applications of the present disclosure.

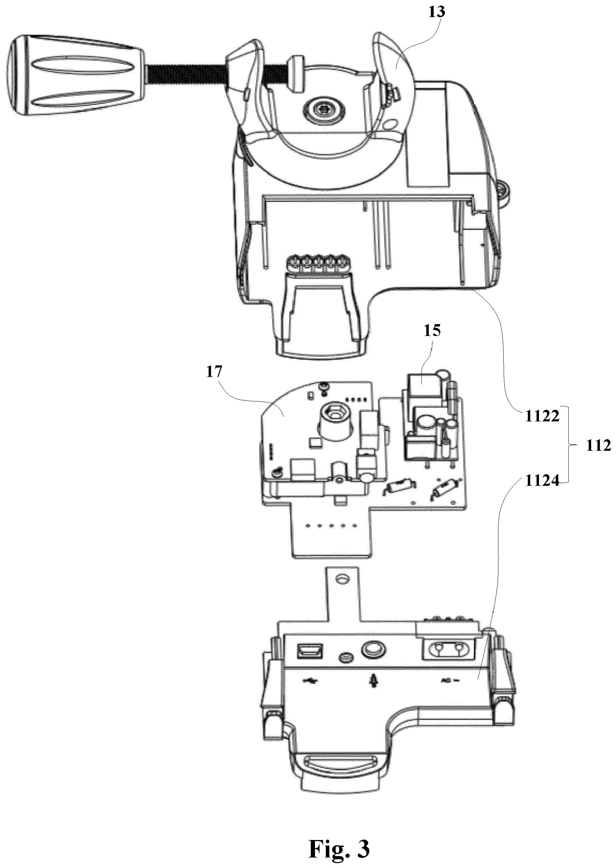

Figures 1 to 3 generally show the structure of the holding device for the liquid infusion pump according to one preferred embodiment of the present disclosure. Figure 1 shows a top view of the holding device. Figure 2 shows a bottom view of the holding device in Figure 1. Figure 3 shows an exploded view of the holding device in Figure 1.

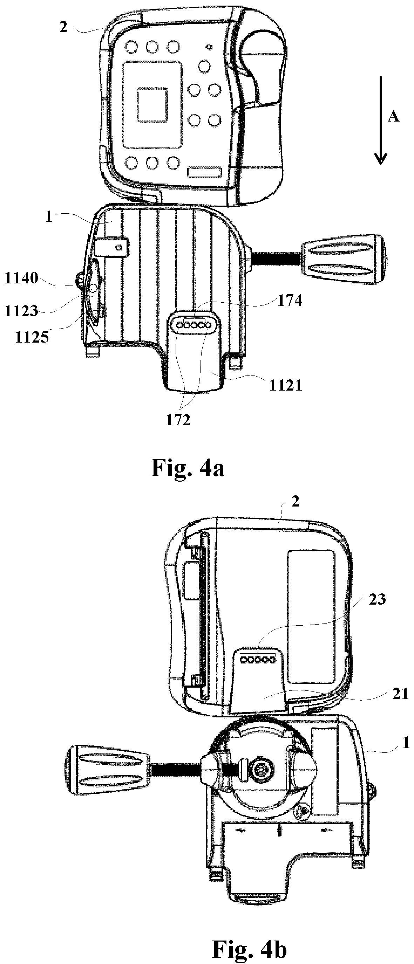

Referring to Figure 1 to Figure 3, holding device 1 for liquid infusion pump 2 according to one preferred embodiment of the present disclosure generally includes a base 11 and a holding member 13. The base 11 is configured to be detachably connected to a liquid infusion pump 2 (shown in Figure 4a to Figure 4e), so as to supply power to the liquid infusion pump 2 and electronically communicate with the liquid infusion pump 2 for real-time monitoring of the infusion operation of the liquid infusion pump 2. Specifically, the base 11 includes a housing 112. The housing 112 generally includes a first housing portion 1122 and a second housing portion 1124. The housing 112 is internally integrated with a power supply adapter 15, a circuit board 17 and the like. The power supply adapter 15 is preferably arranged (e.g., by welding, bonding, screw fixing, snap fixing, etc.) on the circuit board 17 and electrically connected with the circuit board 17. As shown, a power supply interface 152 is provided on the second housing portion 1124. The power supply interface 152 is configured to connect an external alternating current power supply cable and electrically connect with the power supply adapter 15, and the circuit board 17 is thereby supplied with required direct current power supply through the power supply interface 152 and the power supply adapter 15; that is, the power supply adapter 15 converts external alternating current into direct current and supplies the direct current to the circuit board 17. Preferably, means for providing overload protection to the adapter 15 (not shown) is provided in the base 11. Moreover, multiple electronic interfaces for electrically connecting with the liquid infusion pump 2 are provided on the first housing portion 1122. The electronic interfaces are electrically connected with the circuit board 17. The electronic interfaces include first electronic interfaces 172 configured to supply power to the liquid infusion pump 2 and second electronic interfaces 174 configured to carry electronic communication between the base 11 and the liquid infusion pump 2. The base 11 can acquire operating data of the liquid infusion pump 2 through the second electronic interfaces 174. On the other hand, two communication interfaces 176 for connecting communication cables are further provided on the second housing portion 1124. The two communication interfaces 176 are also electrically connected with the circuit board 17, such that the operating data of the liquid infusion pump 2 obtained by the base 11 can be transmitted to an external control and operating device (not shown) via the communication cables. For example, when a patient triggers a call button on the liquid infusion pump 2, preferably, one of the two communication interfaces 176 can transmit call operating data to the external control and operating device, and the other continuously transmits real-time operating parameters of the liquid infusion pump 2 (e.g., infusion pump pressure, infusion rate, time, etc.) to the external control and operating device for monitoring of the real-time operation of the liquid infusion pump 2.

As shown, the holding member 13 is arranged on the housing 112 and is configured to position the base 11 in a predetermined position in service environment. Specifically, the holding member 13 includes a retaining member 132 and an adjusting handle 134 as shown. As shown, the retaining member 132 includes a bottom base 1321 and two retaining arms 1323. The bottom base 1321 is fixed to the housing 112 of the base 11 by screws. The adjusting handle 134 has a thread segment 1341. The thread segment 1341 is threadedly connected with one retaining arm 1323 of the retaining member 132, such that the thread segment 1341 can be displaced relative to the retaining arm 1323 by rotating the adjusting handle 134, thereby a distance between an end portion 1343 of the thread segment 1341 and the other retaining arm 1323 opposite thereto is adjusted to retain, for example, a bed bracket or the like between the end portion 1343 and the other retaining arm 1323 opposite thereto, whereby the holding member 13 can be retained and fixed at the bed bracket or any other predetermined position that can be retained by the holding member 13, and thus the base 11 and the liquid infusion pump 2 carried by the base 11 are fixed in place. It is contemplated that the holding member 13 is not limited to the particular configuration described above, but can be any other suitable configuration known to those of ordinary skills in the art.

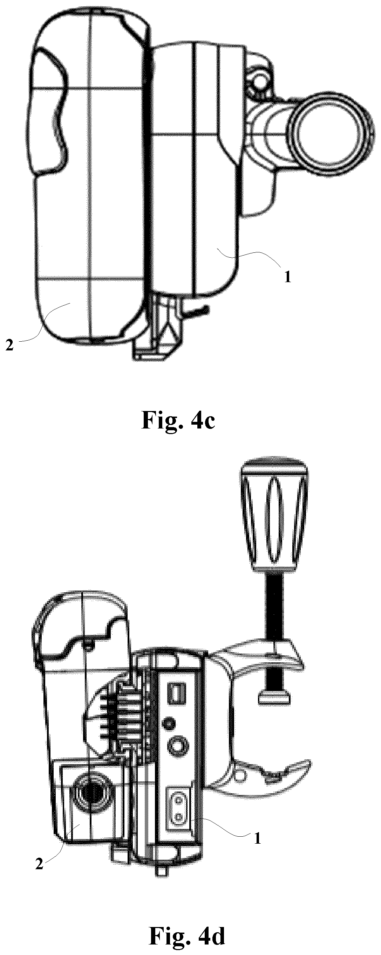

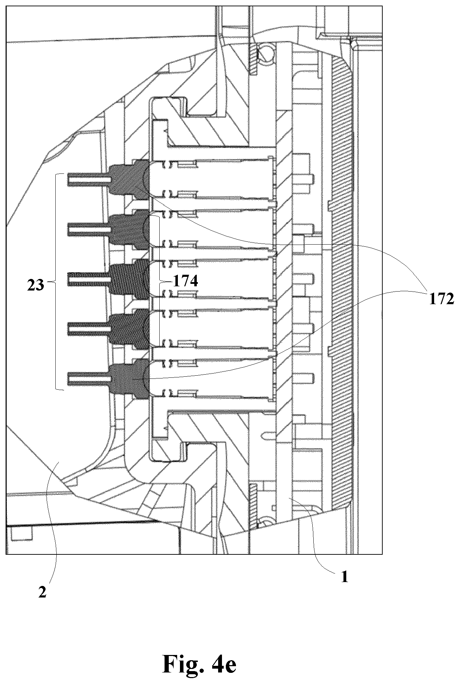

Figure 4a to Figure 4e show the state when the holding device 1 for the liquid infusion pump 2 in Figure 1 is used together with the liquid infusion pump 2. As described above, the base 11 is configured to detachably hold the liquid infusion pump 2, supply power to the liquid infusion pump 2 and electronically communicate with the liquid infusion pump 2 for real-time monitoring of the infusion operation of the liquid infusion pump 2. Preferably, one side of the housing 112 of the base 11 contacts the liquid infusion pump 2 and is detachably attached to the liquid infusion pump 2. In particular, as shown in Figure 4a and Figure 4b, a surface of the housing 112 facing the liquid infusion pump 2 includes a convex portion 1121, and preferably, the plurality of electronic interfaces on the base 11 as described above , i.e., the first electronic interfaces 172 configured to supply power to the liquid infusion pump 2 and the second electronic interfaces 174 configured to carry electronic communication between the base 11 and the liquid infusion pump 2 are provided on the convex portion 1121. Accordingly, a concave portion 21 is provided in a region of the liquid infusion pump 2 that corresponds to the convex portion 1121, and the concave portion 21 is provided with a plurality of electronic interfaces 23 for connecting with the plurality of electronic interfaces on the base 11 in a one-to-one correspondence. Preferably, the size and shape of the concave portion 21 are matched with those of the convex portion 1121, such that the convex portion 1121 can be embedded in the concave portion 21, and such that the plurality of electronic interfaces of the base 11 can be in one-to-one correspondence with and electrically connected with the plurality of electronic interfaces 23 of the liquid infusion pump 2 when the liquid infusion pump 2 is attached to the holding device 1 (as shown in Figure 4d and Figure 4e).

Also, as shown in Figure 4a, the surface of the housing 112 facing the liquid infusion pump 2 further includes a locking rod 1123 having a protrusion 1125 thereon. The protrusion 1125 can be snapped into a corresponding groove (not shown) of the liquid infusion pump 2 so as to position the liquid infusion pump 2. In particular, inside the locking rod 1123, there is a support 1140 and a spring (not shown). The support 1140 is preferably located at the center inside the locking rod 1123 (as shown schematically in dotted line) to provide a pivot point for the locking rod 1123. The spring wraps around the support 1140 and is configured to bias the locking rod 1123 to a free state as shown. As shown in Figure 4a, when the holding device 1 is attached to the liquid infusion pump 2, the holding device 1 and the liquid infusion pump 2 are adjacent to each other and relatively slide close to each other according to the direction indicated by an arrow A in figure 4a, such that the convex portion 1121 of the base 11 is embedded in the concave portion 21 of the liquid infusion pump 2; meanwhile, the protrusion 1125 on the locking rod 1123 will snap into the corresponding groove of the liquid infusion pump 2, and thus the base 11 and the liquid infusion pump 2 are attached to each other. During the process of the projection 1125 on the locking rod 1123 being snapped into the groove of the liquid infusion pump 2, as the holding device 1 slides close to the liquid infusion pump 2, a side wall of the liquid infusion pump 2 approaches and abuts the protrusion 1125 on the locking rod 1123 and pushes the locking rod 1123 against the biasing force of the spring in the locking rod 1123 so as to cause the locking rod 1123 to pivot about the support and deviate from the free position shown in the figures, and until the protrusion 1125 on the locking rod 1123 snaps into the groove of the liquid infusion pump 2, the locking rod 1123 pivots back to the free position shown in the figures under the biasing force of the spring, and is kept fixedly snapped in the groove of the liquid infusion pump 2 by means of the biasing force of the spring. When it is desired to remove the liquid infusion pump 2, it is only necessary to first press the other end of the locking rod 1123 opposite to the protrusion 1125 to deflect the locking rod 1123 away from the free position shown in the figures until the protrusion 1125 on the locking rod 1123 is disengaged from the groove of the liquid infusion pump 2, and the liquid infusion pump 2 is then removed in the reverse direction of the arrow A.

By integrating the power supply adapter 15 into the base 11 of the holding device 1, the base 11 and the liquid infusion pump 2 may be supplied power via only common power supply wires connected to the base 11, and the operation of the device may be ensured by only replacing power supply wires. Therefore, the holding device for the liquid infusion pump and the liquid infusion device including the holding device according to the present disclosure have an optimized configuration and higher cost-effectiveness, and are simple in structure.

In another aspect, since the electronic components, the circuit board and the like inside the base 11 need to be kept dry, the housing 112 of the base 11 needs to be waterproof. In view of the fact that the liquid infusion pump 2 is usually fixed to the bed bracket, and the liquid infusion pump 2 and the holding device 1 are usually fixed in a vertical orientation (in the gravity direction) as shown in Figure 4c or deflected by about -60° to +60° with respect to the vertical direction, the waterproof design for the housing 112 of the base 11 is primarily directed to liquid dropping from the vertical above. Therefore, in the present disclosure, as shown in Figure 3, the housing 112 is designed to include the first housing portion 1122 and the second housing portion 1124 which are constructed as a drawer-type insertion configuration; that is, a portion of the second housing portion 1124 is inserted into the first housing portion 1122 and abuts against the inner wall of the first housing portion 1122 to form a seal. Preferably, the first housing portion 1122 and the second housing portion 1124 are constructed such that in use, the first housing portion 1122 is always located above the second housing portion 1124 in a gravity direction. More preferably, the division of the first housing portion 1122 and the second housing portion 1124 is performed in a direction transverse to the common vertical orientation (in the gravity direction) as shown in Figure 4c. With such a configuration, the dropping liquid can be effectively prevented from penetrating into the sealing seam between the first housing portion 1122 and the second housing portion 1124, and thus remarkably improve the waterproof performance of the housing 112 of the base 11.

Preferably, the first housing portion 1122 is detachably fixed to the second housing portion 1124 by snap engagement or screw connection.

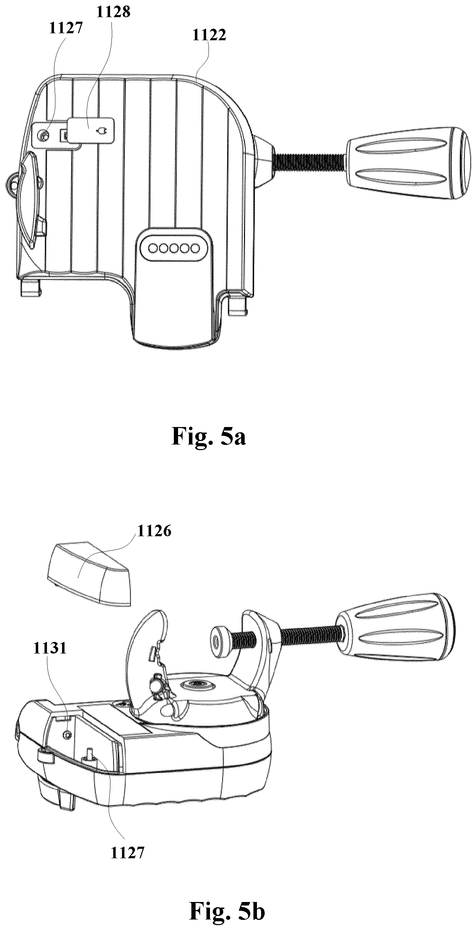

In a further aspect, the housing 112 of the holding device 1 of the present disclosure has a configuration that prevents the user from arbitrarily disassembly. The structure and connection of the housing 112 will be described in detail below with reference to Figure 5a to Figure 5d. Figure 5a to Figure 5d show an exploded schematic view of the holding device 1 for the liquid infusion pump 2 in Figure 1. First, as shown in Figure 5b, the housing 112 includes a side cover 1126 in addition to the first housing portion 1122 and the second housing portion 1124. The side cover 1126 is fixed to the first housing portion 1122 by a side cover screw 1127 located on the back of the first housing portion 1122 shown in Figure 5a. The side cover screw 1127 is covered by a cover plate 1128. The cover plate 1128 is preferably an indicator light panel. The cover plate 1128 is preferably made of a material having elasticity such as rubber, plastic, or the like and is fixed to the back of the first housing portion 1122 by bonding, snapping, or the like. The cover plate 1128 preferably covers the power supply indicator light and has a transparent portion for the light source to pass through. When the side cover 1126 is fixed to the first housing portion 1122 by the side cover screw 1127, the side cover 1126 covers another screw, i.e., a first screw 1131. The first screw 1131, as shown in Figure 5b, connects and fixes the first housing portion 1122 and the second housing portion 1124 together. By arranging the side cover screw 1127 covered by the cover plate 1128 and the first screw 1131 covered by the side cover 1126 as described above, the screws can be better hidden so as to prevent the exposed screws from being easily open due to misoperation, which may result in that the integrity of the housing 112 is damaged and cannot be properly reassembled, and thus the sealing property of the housing 112 is lowered or the components inside the housing 112 are damaged.

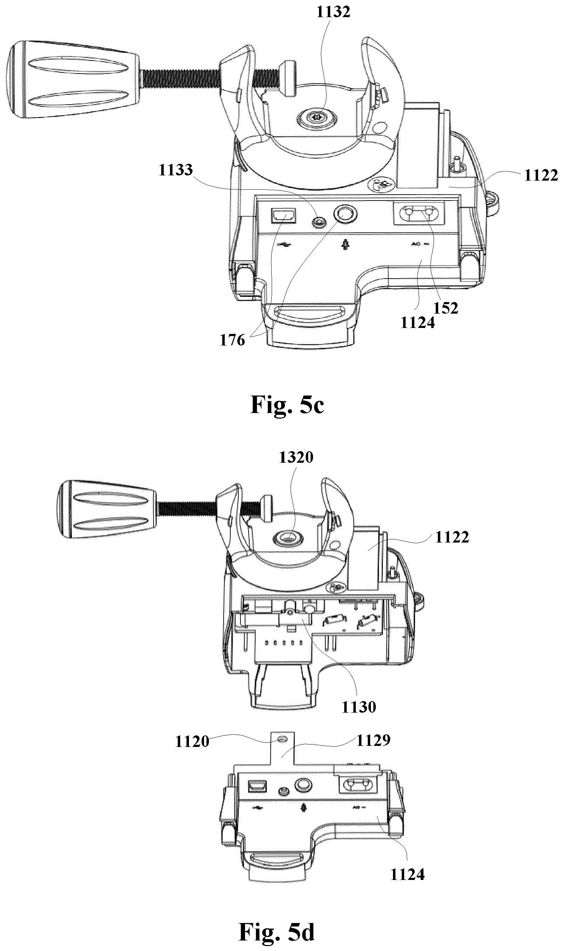

Furthermore, as shown in Figure 5c and Figure 5d, two exposed screws 1132, 1133 are further included to connect and fix the holding member 13, the first housing portion 1122 and the second housing portion 1124 together. Specifically, as shown in Figure 5d, the second housing portion 1124 includes an insertion sheet 1129 which is inserted into the first housing portion 1122. The insertion sheet 1129 in its end portion includes a thread hole 1120. The first housing portion 1122 includes a hole (not shown) that corresponds to the thread hole 1120. The bottom base 1321 of the holding member 13 includes a central hole 1320, such that a second screw 1132 can pass through the central hole 1320 of the bottom base 1321, the above-mentioned hole of the first housing portion 1122, and the thread hole 1120 of the insertion sheet 1129 to fix the bottom base 1321 of the holding member 13 and the second housing portion 1124 (insertion sheet 1129) to the first housing portion 1122. In addition, a third screw 1133, as shown in Figure 5d, fixedly connects the second housing portion 1124 to an inner wall 1130 of the first housing portion 1122 opposite to the second housing portion 1124.

When the housing 112 of the base 11 is detached, the cover plate 1128 on the back of the first housing portion 1122 is firstly removed. The side cover screw 1127 is then loosed to remove the side cover 1126 so as to expose the first screw 1131. The first screw 1131, the second screw 1132, and the third screw 1133 are then loosed, thereby completely detaching the housing 112. When the housing 112 is assembled, the above steps may be performed in reverse order.

The housing 112 designed as described above, and in particular, the design in which the first screw 1131 being hidden can be prevented from being easily detached by the user, such that the use safety, the stability and the water-tightness of the base 11 are further improved.

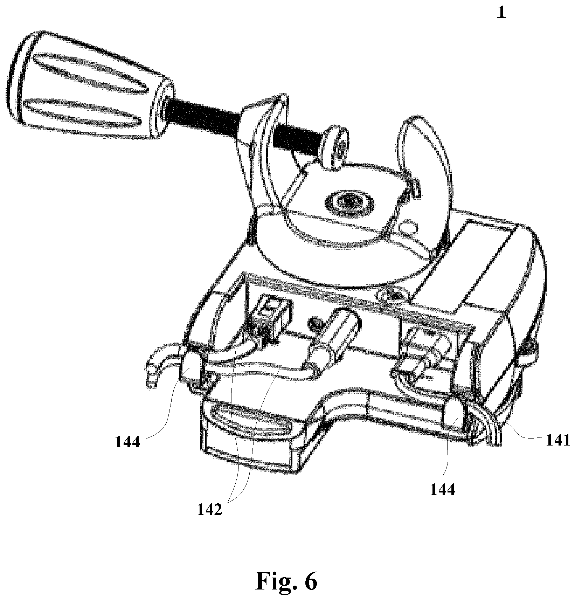

Furthermore, Figure 6 shows the state when a power supply cable 141 and a communication cable 142 are inserted into the holding device 1 for the liquid infusion pump 2 in Figure 1. Preferably, the housing 112 includes two cable holding members 144, which are preferably protruded from the housing 112 to form hook-shaped members as shown in Figure 6, for fixing cables so as to prevent loosen and displacement of cables and prevent external forces applied to the cables from being transmitted to cable plugs which may cause poor contact. It should be understood that the number, positioning and shape of the cable holding members 144 can be modified and adjusted according to actual applications, as long as the cables can be maintained and positioned to prevent loosen and displacement of cables.

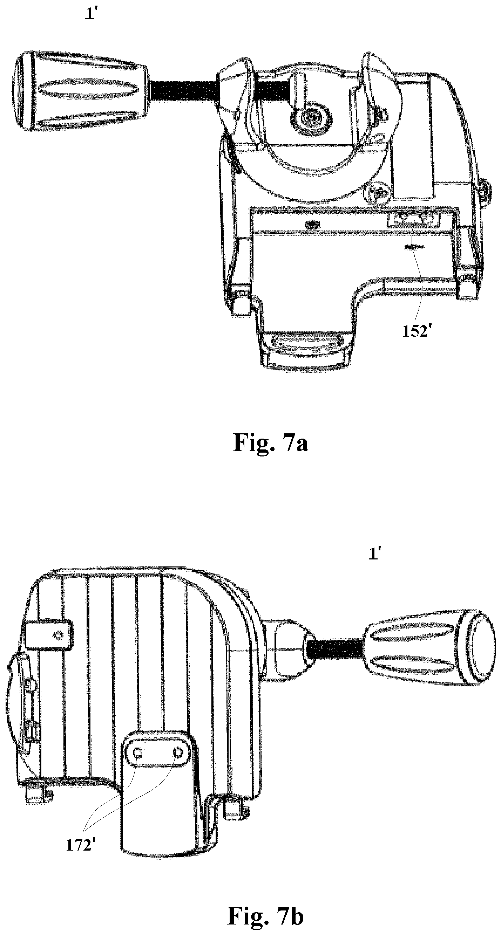

In addition, in the foregoing preferred embodiment according to the present disclosure, the base 11 includes the first electronic interfaces 172 configured to supply power to the liquid infusion pump 2 and the second electronic interfaces 174 configured to carry electronic communication between the base 11 and the liquid infusion pump 2; the base 11 can acquire operating data of the liquid infusion pump 2 through the second electronic interfaces 174; moreover, two communication interfaces 176 for connecting communication cables are further provided on the second housing portion 1124; the two communication interfaces 176 are also electrically connected with the circuit board 17, such that the operating data of the liquid infusion pump 2 obtained by the base 11 can be transmitted to an external control and operating device (not shown) via the communication cables. However, the present disclosure is not limited to this. For example, Figure 7a and Figure 7b respectively show a bottom view and a top view of a holding device 1' for a liquid infusion pump according to another preferred embodiment of the present disclosure. Figure 7a shows a top view of the holding device 1'. Figure 7b shows a bottom view of the holding device 1'.

As shown in Figure 7a and Figure 7b, the holding device 1' for the liquid infusion pump according to another preferred embodiment of the present disclosure has substantially the same configuration as the holding device 1 of Figure 1. The only difference is that the holding device 1' does not have the second electronic interfaces 174 configured to carry electronic communication between the base and the liquid infusion pump 2 and the corresponding two communication interfaces 176 for connecting communication cables of the holding device 1 in Figure 1; the holding device 1' only includes a power supply interface 152' for connecting an external power supply cable and two first electronic interfaces 172' for supplying power to the liquid infusion pump 2. Therefore, in this embodiment, the base 11' is only configured to supply power to the corresponding liquid infusion pump without the effect of transmitting communication. In particular, at this time, the holding device 1' can still be used for the liquid infusion pump 2 as described in the foregoing embodiment, although the liquid infusion pump 2 has the corresponding electronic interfaces for connecting with the second electronic interface 174 of the foregoing holding device 1. When the holding device 1' without the second electronic interface 174 is adopted, it is also possible to keep the electronic interfaces for communication of the liquid infusion pump 2 unconnected, and the liquid infusion pump 2 is kept only electrically connected with the two first electronic interfaces 172' of the holding device 1'.

Furthermore, the holding device according to the disclosure can be applied to various types of liquid infusion pumps, and is not limited to the particular type of liquid infusion pump described.

From the above, it is known that a liquid infusion device according to the present disclosure includes the holding device in each embodiment as described above and various types of liquid infusion pumps.

Various embodiments may be further delineated by combining different embodiments and various technical features in different ways or modifying them, all of which are within the scope of the present disclosure.

The holding device for the liquid infusion pump and the liquid infusion device including the holding device according to the preferred embodiments of the present disclosure have been described above in connection with the detailed description. It is to be understood that the foregoing description is only exemplary and not restrictive. Many variations and modifications will occur to those skilled in the art in light of the above description without departing from the scope of the disclosure. These variations and modifications are also included in the scope of protection of the present disclosure.

## Claims

1. A holding device (1,1') for a liquid infusion pump (2), **characterized in** comprising
a base (11,11') and a holding member (13), wherein
the base (11,11') comprises
a housing (112), to which the liquid infusion pump is detachably attached;
a power supply adapter (15) arranged in the housing (112);
a power supply interface (152,152') configured to electrically connect an external power supply with the power supply adapter (15); and
electronic interfaces configured to electrically connect with the liquid infusion pump (2); and
the holding member (13) is connected with the base (11,11') and is configured to detachably position the holding device (1,1') in a predetermined position in service environment.

2. The holding device (1,1') for the liquid infusion pump (2) according to claim 1, **characterized in that**, the electronic interfaces comprise first electronic interfaces (172,172') configured to supply power to the liquid infusion pump (2).

3. The holding device (1,1') for the liquid infusion pump (2) according to claim 1, **characterized in that**, the electronic interfaces comprise second electronic interfaces (174) configured to carry electronic communication between the base (11,11') and the liquid infusion pump (2).

4. The holding device (1,1') for the liquid infusion pump (2) according to claim 1, **characterized in that**, the housing (112) comprises a first housing portion (1122) and a second housing portion (1124), the first housing portion (1122) and the second housing portion (1124) are configured as a drawer-type insertion configuration, and an edge outer wall of the second housing portion (1124) is lapped jointed to an inner wall of the first housing portion (1122) to form a seal.

5. The holding device (1,1') for the liquid infusion pump (2) according to claim 4, **characterized in that**, the first housing portion (1122) is detachably connected to the second housing portion (1124) by snap engagement or screw connection, and the first housing portion (1122) and the second housing portion (1124) are configured such that, during using, the first housing portion (1122) is always located above the second housing portion (1124) in a gravity direction.

6. The holding device (1,1') for the liquid infusion pump (2) according to claim 4, **characterized in that**, the housing (112) further comprises a side cover (1126), which is fixed to the housing (112) by a side cover screw (1127), and the side cover (1126) is configured to cover a first screw (1131) that connects the first housing portion (1122) and the second housing portion (1124).

7. The holding device (1,1') for the liquid infusion pump (2) according to claim 6, **characterized in that**, the housing (112) further comprises a cover plate (1128) fixed to the housing (112) by bonding or snapping, and the cover plate (1128) is configured to cover the side cover screw (1127).

8. The holding device (1,1') for the liquid infusion pump (2) according to claim 4, **characterized in that**, the second housing portion (1124) comprises an insertion sheet (1129), which is inserted into the first housing portion (1122) and fixed to the first housing portion (1122) by a second screw (1132).

9. The holding device (1,1') for the liquid infusion pump (2) according to claim 8, **characterized in that**, the holding member (13) is positioned on an outer surface of the first housing portion (1122) and fixed to the first housing portion (1122) and the insertion sheet (1129) by the second screw (1132).

10. The holding device (1,1') for the liquid infusion pump (2) according to any of claims 1 to 9, **characterized in that**, the housing (112) comprises cable holding members (144) protruding from the housing (112) to form hook-shaped members, and the cable holding members (144) are configured to fix cables and prevent the cables from loosening and displacement.

11. A liquid infusion device, **characterized in** comprising
a holding device for a liquid infusion pump according to any of claims 1 to 10; and
the liquid infusion pump, which is detachably held by the holding device, wherein
the holding device comprises a base, the base and the liquid infusion pump are fixedly connected to each other by snap-engagement, and electronic interfaces on the base are electrically connected with corresponding electronic interfaces (23) on the liquid infusion pump in a one-to-one correspondence.
